# EUROPEAN PATENT APPLICATION

(11) **EP 4 725 513 A1**
(43) Date of publication of application: **15.04.2026**
(21) Application number: 24819526.5
(22) Date of filing: 28.05.2024
(51) Int. Cl.: A61L 27/02, A61L 27/12, A61L 27/36, A61L 27/56

(54) **BONE GRAFT COMPOSITION AND MANUFACTURING METHOD THEREOF**

(30) Priority: 09.06.2023 KR 20230073910; 13.05.2024 KR 20240062642
(71) Applicant: MediValue Co., Ltd., Seoul 07235 (KR)
(72) Inventor: RHO, Hyung-tay, Seoul 07333 (KR)
(74) Representative: Dehns
(86) International application number: PCT/KR2024/007251
(87) International publication number: WO 2024/253374

(57) **Abstract**

The present invention relates to a bone graft composition comprising mineral trioxide aggregates (MTA) and a porous synthetic bone graft material. The bone graft composition of the present invention can improve the operability of the bone graft composition and form a high-strength structure by adding MTA.

## Description

### Technical Field

The present invention relates to a bone grafting material composition and a method for producing the same, and more particularly, to a bone grafting material composition having improved handleability to easily perform a medical procedure and a method for producing the same.

### Background Art

In order to increase the success rate of implant placement, sufficient bone density is required. However, in some patients, alveolar bone has low bone density due to old age, diseases, or congenital factors, or in severe cases, there is a lack of bone. In such cases, implant placement is performed after sufficiently increasing the bone density using a bone grafting material. The bone grafting material effectively serves as a support during a bone regeneration process to assist in organic and systematic division of blood vessels, tissue cells, and osteoblasts, and is mainly produced in the form of granular powder.

In order for a synthetic bone grafting material to function successfully, various conditions should be satisfied. Among these conditions, the porosity of the synthetic bone grafting material is one of important factors for promoting revascularization, healing, and remodeling of bone. That is, if the porosity is high, the surface area is large to facilitate reabsorption; however, if the porosity is too low, the reabsorption period becomes excessively long, which negatively affects bone regeneration. On the other hand, the higher the porosity, the lower the particle strength of the bone grafting material, and thus it is difficult to maintain the volume of the bone grafting material when external physical pressure is applied after medical procedure of the bone grafting material, and bone formation is not smoothly carried out.

Conventional synthetic bone grafting materials have poor blood permeability, making it difficult for proteins in plasma to be adsorbed, which is unfavorable for bone formation. Therefore, there is a need for the development of a synthetic bone grafting material that has a certain or higher particle strength while having high porosity.

Meanwhile, all bone grafting materials used in dentistry are produced and distributed in the form of powder. Therefore, in order to use conventional bone grafting materials, the surrounding gum covering the alveolar bone defect requiring bone formation is first incised, and then in a state in which a saline solution is mixed with a powdery bone grafting material to make the bone grafting material have an appropriate viscosity, an appropriate amount of the bone grafting material is filled into the alveolar bone defect to form the approximate shape of the alveolar bone where bone regeneration will occur. Subsequently, the bone grafting material is covered with a barrier film, and the incised gum is sutured again.

Therefore, the operation of forming the approximate shape of the alveolar bone where bone regeneration will occur by using the bone grafting material that has been mixed to have an appropriate viscosity is not only quite difficult and time-consuming even for skilled doctors, but also results in significant differences in surgical outcomes depending on the clinical ability of the doctor performing the surgery.

Furthermore, since the volume and shape of the alveolar bone defect requiring bone formation vary from patient to patient, it is very difficult even for skilled doctors to aesthetically form the shape of the alveolar bone where bone regeneration will occur to match the left and right symmetry of the patient's maxilla or mandible by using the bone grafting material that has been mixed to have an appropriate viscosity.

### Disclosure of Invention

### Technical Problem

An object of the present invention is to solve the above problems, and to provide a bone grafting material composition of which the handleability is improved by mixing mineral trioxide aggregates (MTA) with a porous synthetic bone grafting material, and which is capable of forming a high-strength structure.

Another object of the present invention is to provide a method for producing a bone grafting material composition, the method being capable of improving strength by mixing a porous synthetic bone grafting material and MTA.

### Solution to Problem

According to an aspect of the present invention, there is provided a bone grafting material composition including mineral trioxide aggregates (MTA) and a porous synthetic bone grafting material.

The MTA may comprise tricalcium silicate (C₃S) and/or dicalcium silicate (C₂S).

The MTA may comprise only tricalcium silicate (C₃S), or may comprise a mixture of tricalcium silicate (C₃S) and dicalcium silicate (C₂S) in a weight ratio of 8:2 to 2:8.

The porous synthetic bone grafting material may comprise hydroxyapatite (HA) and/or beta-tricalcium phosphate (β-TCP).

The porous synthetic bone grafting material may comprise hydroxyapatite (HA) and beta-tricalcium phosphate (β-TCP) in a weight ratio of 9:1 to 1:9.

The bone grafting material composition may comprise a porous synthetic bone grafting material coarse powder and/or a porous synthetic bone grafting material fine powder.

The porous synthetic bone grafting material coarse powder may have an average particle size of 0.1 to 1.0 mm.

The porous synthetic bone grafting material fine powder may have an average particle size of 0.05 to 0.1 mm.

The bone grafting material composition may comprise 20 to 70 parts by weight of the MTA with respect to 100 parts by weight of the porous synthetic bone grafting material.

In the bone grafting material composition, with respect to 100 parts by weight of the MTA, only the coarse powder may be comprised, or the coarse powder and the fine powder may be mixed at a weight ratio of 9:1 to 5:5.

According to another aspect of the present invention, there is provided a method for producing a bone grafting material composition, the method including: (a) a step of preparing a porous synthetic bone grafting material having pores; (b) a step of preparing MTA; and (c) a step of mixing the porous synthetic bone grafting material and the MTA to produce a bone grafting material composition.

In the step (a), the porous synthetic bone grafting material may be pulverized to have different average particle sizes, thereby producing a porous synthetic bone grafting material coarse powder and/or a porous synthetic bone grafting material fine powder.

### Advantageous Effects of Invention

A bone grafting material composition according to the present invention has improved handleability by adding mineral trioxide aggregates (MTA), and is capable of forming a high-strength structure.

A method for producing a bone grafting material composition according to the present invention is capable of improving strength by mixing MTA and synthetic bone grafting materials having different average particle sizes.

### Brief Description of Drawings

Fig. 1 is a schematic diagram illustrating a bone grafting material composition according to the present invention.
Fig. 2 is a photograph showing changes in viscosity and handleability after mixing bone grafting materials produced according to Examples of the present invention with water.
Fig. 3 is a photograph showing states after bone grafting materials produced according to Examples of the present invention are mixed with water and hardened.
Fig. 4 is a photograph showing a fracture surface, taken by using a scanning electron microscope, after bone grafting material compositions produced according to Examples of the present invention are mixed with water and hardened.
Fig. 5 is a graph showing results of measuring a setting time after bone grafting material compositions produced according to Examples of the present invention are mixed with water and hardened.
Fig. 6 is a photograph showing a hardened state after bone grafting material compositions produced according to Examples of the present invention are mixed with water and hardened.
Fig. 7 is a graph showing results of measuring the compressive strengths of bone grafting material compositions produced according to Examples of the present invention.
Fig. 8 is a photograph showing the degree of wettability by confirming penetration after filling a red pigment-added saline solution to bone grafting material compositions produced according to Examples of the present invention.
Fig. 9 shows results of measuring a contact angle after dropping a water droplet on the surface of bone grafting material compositions produced according to Examples of the present invention.

### Best Mode for Carrying out the Invention

Hereinafter, the present invention will be described in detail. In describing the present invention, detailed descriptions of well-known components or functions may be omitted.

The terms or words used in this specification and claims are not limited to their ordinary or dictionary meanings, but should be interpreted as having meanings and concepts that conform to the technical matter of the present invention.

Embodiments described in this specification and the configurations illustrated in the drawings are preferred embodiments of the present invention and do not represent all of the technical concepts of the present invention. Therefore, various equivalents and modifications that can replace these are possible at the filing of this application.

Hereinafter, a bone grafting material composition according to the present invention will be described in detail.

The bone grafting material composition according to the present invention comprises mineral trioxide aggregates (MTA) and a porous synthetic bone grafting material.

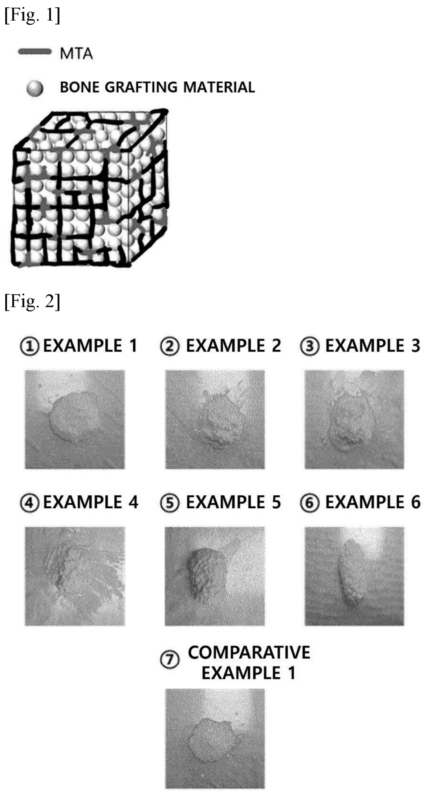

Fig. 1 is a schematic diagram illustrating a bone grafting material composition according to the present invention.

Referring to Fig. 1, the MTA may crosslink the porous synthetic bone grafting material and form a high-strength structure of the bone grafting material (matrix scaffolding). This makes it possible to maintain a structure that does not deform even when pressed by soft tissues, and maintain the volume of the bone grafting material. Furthermore, due to the hydro-curing characteristics of the MTA, the viscosity of the bone grafting material increases to improve handleability (agglomeration, formability, etc.), thereby providing an advantage in allowing a practitioner to perform a medical procedure easily.

The MTA may comprise tricalcium silicate (C₃S) and/or dicalcium silicate (C₂S), and preferably, may comprise only tricalcium silicate (C₃S) or may comprise a mixture of tricalcium silicate (C₃S) and dicalcium silicate (C₂S) in a weight ratio of 8:2 to 2:8.

The bone grafting material composition may comprise coarse powder and fine powder of porous synthetic bone grafting material powder with different average particle sizes, rather than porous synthetic bone grafting material powder pulverized to have a single average particle size.

The average particle size of the porous synthetic bone grafting material powder with different average particle sizes may be 0.05 to 1.0 mm.

The average particle size of the porous synthetic bone grafting material coarse powder may be 0.1 to 1.0 mm.

The average particle size of the porous synthetic bone grafting material fine powder may be 0.05 to 0.1 mm.

The porous synthetic bone grafting material is obtained by mixing coarse powder and/or fine powder with various average particle sizes rather than powder with a single average particle size, and therefore, the spaces between large-particle-size porous synthetic bone grafting materials filled with small-particle-size porous synthetic bone grafting materials, thereby making it possible to form a bone grafting material with higher strength.

The porous synthetic bone grafting material may comprise hydroxyapatite (HA) and/or beta-tricalcium phosphate (β-TCP).

The porous synthetic bone grafting material may comprise hydroxyapatite (HA) and beta-tricalcium phosphate (β-TCP) in a weight ratio of 9:1 to 1:9.

The bone grafting material composition may comprise 20 to 70 parts by weight of the MTA with respect to 100 parts by weight of the porous synthetic bone grafting material.

If the MTA is comprised in an amount less than 20 parts by weight, the MTA may fail to form a high-strength structure, resulting in low strength. If the MTA is comprised in an amount exceeding 70 parts by weight, the amount of the bone grafting material may be too small, resulting in a decrease in bone regeneration rate.

Hereinafter, a method for producing the bone grafting material composition according to the present invention will be described.

First, a porous synthetic bone grafting material including pores is prepared (step a).

The porous synthetic bone grafting material may be pulverized to have different average particle sizes, thereby preparing porous synthetic bone grafting material coarse powder and/or porous synthetic bone grafting material fine powder.

The average particle size of the porous synthetic bone grafting material coarse powder may be 0.1 to 1.0 mm.

The average particle size of the porous synthetic bone grafting material fine powder may be 0.05 to 0.1 mm.

Preferably, the porous synthetic bone grafting material may comprise hydroxyapatite (HA) and beta-tricalcium phosphate (β-TCP) in a weight ratio of 9:1 to 1:9.

Next, the MTA is prepared (step b).

The MTA may comprise tricalcium silicate (C₃S) and/or dicalcium silicate (C₂S), and preferably, may comprise only tricalcium silicate (C₃S), or may comprise a mixture of tricalcium silicate (C₃S) and dicalcium silicate (C₂S) in a weight ratio of 8:2 to 2:8.

The MTA has a powdery form, and the average particle size of the MTA powder may be 10 to 50 µm.

Finally, the porous synthetic bone grafting material and the MTA are mixed to produce a bone grafting material composition (step c).

### Mode for Carrying Out the Invention

Hereinafter, preferred embodiments of the present invention will be described. However, these are for examples only, and the scope of the present invention is not limited thereby.

### <Preparation Example 1> Synthesis of MTA (Calcium Silicate)

High-purity raw material of 81.7g of CaCO₃ (99%, Sigma-Aldrich), 16.9g of SiO₂ (99.9%, High Purity Chemicals), 0.9g of MgO (98%, Sigma-Aldrich), 0.2g of Na₂CO₃ (99.5%, Sigma-Aldrich) and 0.3g of CaF₂ (98%, Jensei) powders were weighed and pure tricalcium silicate (C₃S) was synthesized through a solid-state synthesis method.

Specifically, the weighed powders were mixed using a ball mill in an ethyl alcohol solvent for 24 hours. The mixed powders were heat-treated at 1,450 °C for 7 hours and then cooled. The synthesized C₃S lumps were pulverized using a ball mill or a mortar and pestle to prepare MTA powder.

### <Preparation Example 2> Synthesis of Hydroxyapatite (HA)

59.2g of calcium carbonate (CaCO₃, 99%, Sigma Aldrich) and 40.8g of ammonium phosphate (NH₄H₂PO₄, 99%, Sigma-Aldrich) powders were weighed. Then, alumina balls with a weight ratio of balls and powder of 3.86 were added, and the powders were mixed by ball milling in an ethyl alcohol solvent for 24 hours. The powders were dried at 70 °C for 24 hours.

The dried powders were heat-treated at 1,200 °C for 2 hours and then cooled. The synthesized hydroxyapatite lumps were pulverized using a ball mill or a mortar and pestle to prepare hydroxyapatite powder.

### <Preparation Example 3> Synthesis of Tricalcium Phosphate (TCP)

56.6g of calcium carbonate (CaCO₃, 99%, Sigma Aldrich) and 43.4g of ammonium phosphate (NH₄H₂PO₄, 99%, Sigma-Aldrich) powders were weighed. Then, alumina balls with a weight ratio of balls and powder of 3.86 were added, and the powders were mixed by ball milling in an ethyl alcohol solvent for 24 hours. The powders were dried at 70 °C for 24 hours.

The dried powders were heat-treated at 900 °C for 10 hours and then cooled. The synthesized tricalcium phosphate lumps were pulverized using a ball mill or a mortar and pestle to prepare powder.

### <Preparation Example 4> Preparation of Porous Synthetic Bone Grafting Material

A bone grafting material powder, which is obtained by mixing the HA prepared according to <Preparation Example 2> and the TCP prepared according to <Preparation Example 3> at a mass ratio of 6:4 (wt%), was mixed with distilled water (di-water) at a mass ratio of 5:5 to produce a slurry. A polyurethane sponge (45 ppi) was immersed in the prepared slurry, and dried at 70 °C for 24 hours to cause the produced slurry to be adsorbed on the sponge. The sponge with the slurry adsorbed thereon was heated up to 600 °C for 10 hours, then heated up to 1100 °C for 1 hour and 40 minutes, and then sintered at 1100 °C for 4 hours. The porous synthetic bone grafting material, which has been sintered, was pulverized using a mortar and pestle to prepare porous synthetic bone grafting materials with different average particle sizes.

### <Examples> Production of Bone Grafting Material Composition

The MTA prepared according to <Preparation Example 1> and the porous synthetic bone grafting material prepared according to <Preparation Example 4> were mixed at the ratios shown in Table 1 below to prepare bone grafting material compositions. The porous synthetic bone grafting material prepared according to <Preparation Example 4> in which no MTA was mixed was used as Comparative Example.

**[Table 1]**

| Classification | MTA (wt%) | Porous Synthetic Bone Grafting Material (wt%) | |
|---|---|---|---|
| | | Coarse powder | Fine Powder |
| Example 1 (15MTA-85BG) | 15 | 50 | 35 |
| Example 2 (20MTA-80BG) | 20 | 50 | 30 |
| Example 3 (30MTA-70BG) | 30 | 50 | 20 |
| Example 4 (40MTA-60BG) | 40 | 50 | 10 |
| Example 5 (50MTA-50BG) | 50 | 50 | 0 |
| Example 6 (60MTA-40BG) | 60 | 40 | 0 |
| Comparative Example (100BG) | 0 | 100 | 0 |

### <Experimental Example 1> Handleability Test

Fig. 2 is a photograph showing changes in viscosity and handleability after mixing bone grafting material compositions produced according to Examples with water.

First, 0.5g of the bone grafting material composition produced according to each Example was prepared, and then the bone grafting material composition was mixed with water at a water-to-powder ratio of 0.5:1.

Referring to Fig. 2, in Comparative Example 1 (100BG), the bone grafting material is not mixed with water at all and remains separated, and addition of MTA causes the composition to have a viscosity. It was confirmed that the expression of viscosity becomes more excellent as the MTA content is higher.

In particular, the bone grafting material compositions produced according to Example 5 (50MTA-50BG) and Example 6 (60MTA-40BG) were able to be directly handled by hands. In practice, in Fig. 2, the bone grafting material compositions produced according to Example 5 (50MTA -50BG) and Example 6 (60MTA -40BG) were handled directly, made into a plug shape to be easily applied to a defect site, and then photographed.

### <Experimental Example 2> MTA Structure Formation Test

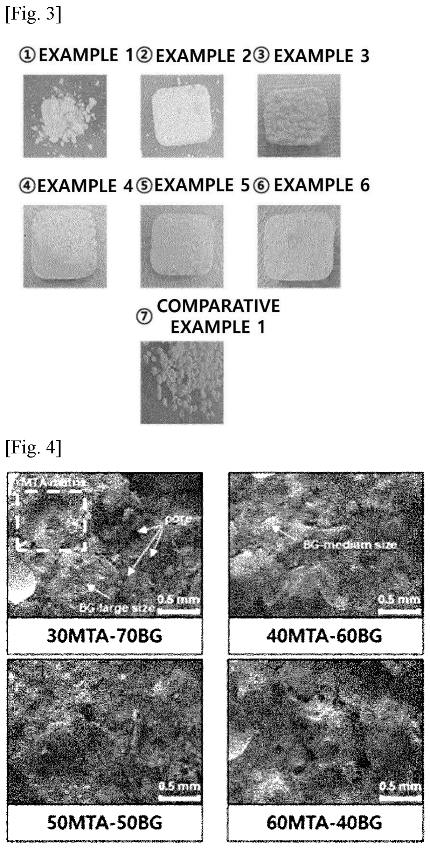

Fig. 3 is a photograph showing the state in which a bone grafting material composition produced according to each Example is mixed with water and hardened, in order to confirm whether the bone grafting material composition maintain its structure after hardening, and Fig. 4 is a photograph, of a fracture surface of the hardened bone grafting material composition, taken by using a scanning electron microscope.

First, 0.5g of a bone grafting material composition produced according to each Example was prepared, and then the bone grafting material composition was mixed with water at a water-to-powder ratio of 0.5:1.

Referring to Fig. 3, it can be seen that in all Examples, the MTA satisfactorily forms a structure between the bone grafting materials. In addition, numerous micropores can be observed in the MTA itself as well.

Referring to Fig. 4, it can be seen that the bone grafting material composition having an MTA content of 15 wt% fails to form a structure and breaks. The bone grafting material composition having an MTA content of 20 wt% maintains its structure at the time of being taken out of the mold, but has very low strength, making it difficult to handle the bone grafting material composition afterwards. When the bone grafting material composition has a MTA content of 30 wt% or more, there was no difficulty in handling, and it can be seen that the surface becomes smoother as the MTA content is higher. In Fig. 4, Examples 1 and 2, in which it was impossible to maintain its structure or difficult to handle the composition after hardening, were excluded from the test.

### <Experimental Example 3> Hardening Test

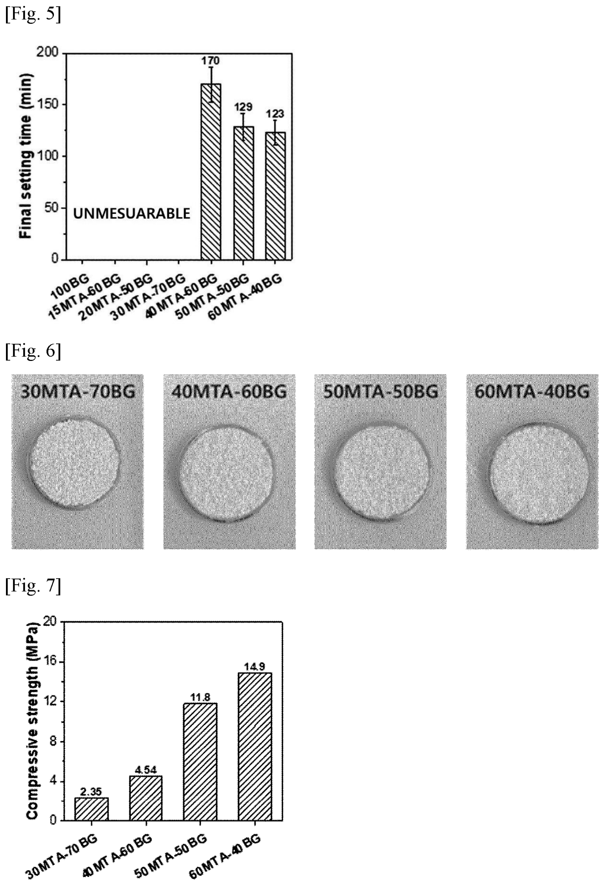

Fig. 5 shows the results of measuring the setting time after mixing a bone grafting material composition produced according to each Example with water.

The hardening test was carried out in accordance with the ISO-6876 standards based on MTA. First, 0.7g of the bone grafting material composition produced according to each Example was prepared, and then the bone grafting material composition was mixed with water at a water-to-powder ratio of 0.5:1. Thereafter, a sample was filled into a dental plaster mold (inner diameter 10 mm, height 2 mm), and a 100g indenter (diameter 2 mm) was vertically applied to the sample over a setting time, and then, measuring the time until no mark (indentation) remained from the mixing point was repeated three times.

Referring to Fig. 5, Comparative Example in which the porous synthetic bone grafting material produced according to Experimental Example 4 had no MTA mixed was not hardened, and in the bone grafting material composition having a MTA content of 30wt% or less, observation of the indentation from MTA was impossible due to a large amount of bone grafting material, and thus the hardening state could not be determined according to this standard as shown in Fig. 6. It was shown that if the MTA content is 40 wt% or more, the setting time decreases as the MTA content increases.

### <Experimental Example 4> Compression Strength Test

Fig. 7 shows the results of a compression strength test for bone grafting material compositions produced according to Examples, which have an MTA content of 30 wt% or more and in which an MTA structure is formed.

First, 1.0g of the bone grafting material composition produced according to each Example was prepared, and then the bone grafting material composition was mixed with water at a water-to-powder ratio of 0.5:1. Thereafter, the mixture was filled into a mold (7 X 7 X 14 mm) and hardened for 24 hours. After removing the mold from the hardened sample, the cross-sectional area of the sample was calculated and tested.

A universal testing machine was used to apply a load to the sample which has been hardened for 24 hours, and the maximum value at the point when the sample was fractured was recorded. The strength (kgf) at the point when the specimen was fractured was divided by the cross-sectional area(cm²) and the result was converted in units of MPa, which is the compressive strength per cross-sectional area.

Referring to Fig. 7, the compressive strength increases as the proportion of MTA increases. In particular, it can be seen that the compressive strength is significantly improved when the MTA content is 50 wt% or more.

Compared with the bone grafting material composition produced according to <Example 3> in which 30 wt% of MTA is added, the bone grafting material composition produced according to <Example 6> in which 60 wt% of MTA is added exhibited a compression strength increased by about 6 times, indicating that MTA forms a high-strength matrix.

### <Experimental Example 5> Wettability Test

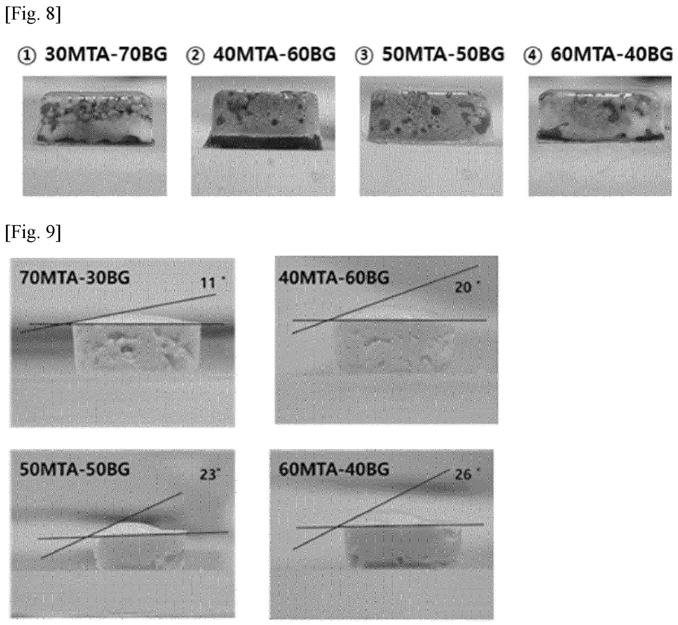

Fig. 8 is the results of measuring contact angles to determine the wettability of the bone grafting material compositions produced according to <Examples 3> to <Example 6>. Fig. 9 is a photograph from which it is possible to confirm whether a material is actually wetted by filling a red pigment-added saline solution to the bone grafting material composition.

Generally, the method of confirming the wettability of a material is performed by measuring the contact angle. A general wettability measurement involves dropping a single droplet of liquid, including water, in a dry state and measuring the angle between the droplet and the surface of a test specimen to determine wettability. However, it was found that the bone grafting material has a porous structure, and thus a water droplet does not form on the surface unless the interior of the bone grafting material is filled with moisture. Therefore, the contact angle measurement in Fig. 8 could not be measured using separate equipment, and the contact angle was measured based on water formed after the interior of the bone grafting material is filled with the water. As a result, the contact angles of the bone grafting material compositions were all recorded as 45° or less, at which a material is generally considered to be wetted.

Referring to Fig. 9, it can be seen with naked eyes that the red pigment actually penetrates smoothly into the bone grafting material composition in all Examples on which the test has been carried out.

## Claims

1. A bone grafting material composition comprising mineral trioxide aggregates (MTA) and a porous synthetic bone grafting material.

2. The bone grafting material composition of claim 1, wherein the MTA comprises tricalcium silicate (C₃S) and/or dicalcium silicate (C₂S).

3. The bone grafting material composition of claim 2, wherein the MTA comprises only tricalcium silicate (C₃S), or comprises a mixture of tricalcium silicate (C₃S) and dicalcium silicate (C₂S) in a weight ratio of 8:2 to 2:8.

4. The bone grafting material composition of claim 1, wherein the porous synthetic bone grafting material comprises hydroxyapatite (HA) and/or beta-tricalcium phosphate (β-TCP).

5. The bone grafting material composition of claim 1, wherein the bone grafting material composition comprises porous synthetic bone grafting material coarse powder and/or porous synthetic bone grafting material fine powder.

6. The bone grafting material composition of claim 5, wherein an average particle size of the porous synthetic bone grafting material coarse powder is 0.1 to 1.0 mm.

7. The bone grafting material composition of claim 5, wherein an average particle size of the porous synthetic bone grafting material fine powder is 0.05 to 0.1 mm.

8. The bone grafting material composition of claim 4, wherein the porous synthetic bone grafting material comprises hydroxyapatite (HA) and beta-tricalcium phosphate (β-TCP) in a weight ratio of 9:1 to 1:9.

9. The bone grafting material composition of claim 1, wherein the bone grafting material composition comprises 20 to 70 parts by weight of the MTA with respect to 100 parts by weight of the porous synthetic bone grafting material.

10. The bone grafting material composition of claim 5, wherein the bone grafting material composition comprises only the porous synthetic bone grafting material coarse powder, or comprises a mixture of the porous synthetic bone grafting material coarse powder and the porous synthetic bone grafting material fine powder in a weight ratio of 9:1 to 5:5.

11. A method for producing a bone grafting material composition, the method comprising:
(a) a step of preparing a porous synthetic bone grafting material having pores;
(b) a step of preparing MTA; and
(c) a step of mixing the porous synthetic bone grafting material and the MTA to produce a bone grafting material composition.

12. The method of claim 11, wherein in the step (a), the porous synthetic bone grafting material is pulverized to have different average particle sizes, thereby producing porous synthetic bone grafting material coarse powder and/or porous synthetic bone grafting material fine powder.
